# EUROPEAN PATENT APPLICATION

(11) **EP 2 179 720 A1**
(43) Date of publication of application: **28.04.2010**
(21) Application number: 08790400.9
(22) Date of filing: 07.08.2008
(51) Int. Cl.: A61K 8/25, A61K 8/31, A61K 8/37, A61K 8/89, A61Q 15/00

(54) **PREPARATION FOR EXTERNAL APPLICATION TO SKIN**

(30) Priority: 20.08.2007 JP 2007213875
(71) Applicant: Shiseido Company, Ltd., Chuo-ku Tokyo 104-8010 (JP)
(72) Inventor: KAMIYA, Yukiko, Yokohama-shi Kanagawa 224-8558 (JP); ARAKI, Hidefumi, Yokohama-shi Kanagawa 224-8558 (JP); YOSHIKAWA, Norinobu, Yokohama-shi Kanagawa 224-8558 (JP)
(74) Representative: Merkle, Gebhard
(86) International application number: PCT/JP2008/002143
(87) International publication number: WO 2009/025071

(57) **Abstract**

The present invention provides an external preparation for skin comprising antibacterial zeolite and a nonvolatile liquid oil component that comprises a nonionic surfactant having a HLB of 6-8 that has a polyoxyethylene chain.

The object of the present invention is to provide an external preparation for skin, particularly a deodorant cosmetic, that enables more effective removal of its stain on clothing by washing.

## Description

### TECHNICAL FIELD

The present invention relates to an external preparation for skin. Even more specifically, it relates to an external preparation for skin that is used, for example, as a deodorizing cosmetic containing antibacterial zeolite and possessing a superior deodorizing effect and improved cleanability of stained clothing due to the antibacterial zeolite.

### BACKGROUND ART

Antibacterial zeolite powder is mainly added, as a deodorant, to external preparations for skin such as deodorizing cosmetics.

For example, compositions for antibacterial sprays (see Patent Citation 1) and deodorizing cosmetics (see Patent Citation 2) containing antibacterial zeolite have been developed.
Furthermore, technology that blends silicone into antibacterial zeolite as a deodorizing cosmetic with improved anti-discoloring properties has been disclosed (see Patent Citation 3).

A deodorizing cosmetic (or antiperspirant cosmetic) is a cosmetic that is used to prevent or control emanation and/or secretion of offensive body odor, or to eliminate the emanated and/or secreted components. In terms of the product form, it is commonly used as a lotion, cream, powder, stick, aerosol, etc.

The deodorizing cosmetics disclosed in Patent Citations 1 to 3 use a deodorizing method utilizing the bactericidal action of antibacterial zeolite. A deodorizing cosmetic using antibacterial zeolite has a shortcoming in that it discolors after application and tends to stain clothing in such a way that stains remain on the clothing even after washing. Therefore, development of a deodorizing cosmetic that has superior discoloration/staining prevention effects, superior sensation during use, and a good deodorizing effect is desired.

On the other hand, in Patent Citation 4 the applicant of the present application disclosed technology for reducing the stain occurring from antibacterial zeolite adhered to clothing by adding polyoxyethylene polyoxypropylene 2-decyltetradecyl ether.

Patent Citation 1: Japanese Patent Laid-0pen S63-250325 bulletin
Patent Citation 2: Japanese Patent Laid-0pen H8-26956 bulletin
Patent Citation 3: Japanese Patent Laid-0pen H8-92051 bulletin
Patent Citation 4: Japanese Patent Laid-0pen 2005-97170 bulletin

### DISCLOSURE OF INVENTION

### TECHNICAL PROBLEM

However, in a system having a large blend ratio of nonvolatile liquid oil components, the addition of polyoxyethylene polyoxypropylene 2-decyltetradecyl ether does not necessarily improve the cleanability of stained clothing due to the antibacterial zeolite.
Therefore, stain reduction can be attempted by increasing its blend ratio. However, a high blend ratio is not preferable from the point of view of the sensation during use.

The inventors conducted earnest research on the aforementioned problem of an external preparation for skin containing antibacterial zeolite and newly discovered that, even for an external preparation for skin having a high blend ratio of nonvolatile liquid oil components (an external preparation for skin having a blend ratio of 10 mass % or more, for example), the cleaning effect on stained clothing by washing improved by adding a nonionic surfactant having a HLB of 6-8 that has a polyoxyethylene chain, thus completing the present invention.
It was also discovered that the addition of powder (preferably porous silica having a specific surface area of 150 m²/g or more) could prevent discoloration of the base agent itself (particularly an oil based base agent) of the external preparation for skin.

The object of the present invention is to provide an external preparation for skin that has a superior effect on the cleaning/removal of stains on clothing by washing and can be utilized preferably as a deodorant cosmetic in particular.

### TECHNICAL SOLUTION

That is, the present invention provides an external preparation for skin comprising antibacterial zeolite and a nonvolatile liquid oil component, said external preparation for skin comprising a nonionic surfactant having a HLB of 6-8 that has a polyoxyethylene chain.

Also, the present invention provides the aforementioned external preparation for skin wherein the blend ratio of said nonionic surfactant having a HLB of 6-8 that has a polyoxyethylene chain is 10 mass % or more of the total amount of the nonvolatile liquid oil component.

Furthermore, the present invention provides the aforementioned external preparation for skin that additionally comprises porous silica having a specific surface area of 150 m²/g or more.

Also, the present invention provides the aforementioned external preparation for skin wherein said nonvolatile liquid oil component is one, two or more selected from a group consisting of liquid petrolatum, cetyl octanoate, methylphenylpolysiloxane, squalane, glyceryl tri-2-ethylhexanoate, glyceryl diisostearate, pentaerythrityl tetra-2-ethylhexanoate, trimethylolpropane trioctanoate, trimethylolpropane triisostearate, and diisostearyl malate.

Furthermore, the present invention provides the aforementioned external preparation for skin that is prepared as an oil based product.

Also, the present invention provides the aforementioned external preparation for skin wherein said external preparation for skin is in the stick form.

### ADVANTAGEOUS EFFECTS

The external preparation for skin of the present invention has a superior effect on the cleaning/removal of stains occurring from its adhesion to clothing. Also, it has a superior effect in terms of prevention of discoloration of the base agent (particularly an oil based base agent) of the external preparation for skin.

### BEST MODE FOR CARRYING OUT THE INVENTION

The present invention is described in detail below.

### <Antibacterial zeolite>

The antibacterial zeolite used in the present invention is zeolite powder that holds antibacterial metal ions in its ion-exchangeable parts; i.e. zeolite powder whose exchangeable ions are partly or entirely replaced by antibacterial metal. In the present invention, zeolite having ammonium ion substitution in addition to antibacterial metal ion substitution is also preferable.

For the zeolite, either natural zeolite or synthetic zeolite can be used. Zeolite is aluminosilicate having a three dimensional skeletal structure; it is represented by the general formula XM_{2/n}O · Al₂O₃ · YSiO₂ · ZH₂O. In this general formula, M denotes an exchangeable ion, usually a monovalent or divalent metal ion. n denotes the atomic valence of the (metal) ion. X and Y denote a metal oxide and the silica factor, respectively, and Z denotes the number of the crystallization water molecules.

Specific examples of zeolite include A-type zeolite, X-type zeolite, Y-type zeolite, T-type, high silica zeolite, sodalite, mordenite, analcime, crinoptyrolite, chabasite, and erionite.

The ion exchange capacity of these zeolites are: 7 meq/g for A-type zeolite, 6.4 meq/g for X-type zeolite, 5 meq/g for Y-zeolite, 3. 4 meq/g for T-type, 11.5 meq/g for sodalite, 2.6 meq/g for mordenite, 5 meq/g for analcime, 2.6 meq/g for crinoptyrolite, 5 meq/g for chabasite, and 3.8 meq/g for erionite. Any of these has enough capacity for ion exchange with antibacterial metal ions and/or ammonium ions.

Examples of exchangeable ions in zeolite include sodium ions, calcium ions, potassium ions, magnesium ions, and iron ions. Examples of the antibacterial metal ions to substitute for these ions include silver, copper, zinc, mercury, tin, lead, bismuth, cadmium, chromium, and thallium ions; preferably silver, copper, or zinc ions, and more preferably silver ions.

The content of the antibacterial ions is preferably 0.1-15 mass % of the zeolite. For example, antibacterial zeolite containing 0.1-15% of silver ion and 0.1-8 mass % of copper ion or zinc ion is preferable. On the other hand, zeolite can contain up to 20 mass % of ammonium ions; however, for the purpose of effectively preventing discoloration of the zeolite, 0.5-5% is preferable and 0.5-2 mass % is more preferable. "mass %" means the mass percentage in 110°C dry standard zeolite.

In the present invention, commercial products can be used for the antibacterial zeolite. The antibacterial zeolite is prepared, for example, as follows. That is, zeolite is exposed to a mixed solution containing antibacterial metal ions such as silver ions, copper ions, and zinc ions, prepared in advance, to substitute the aforementioned ions for the exchangeable ions in the zeolite. The exposure can be achieved by the batch method or continuous method (column method, for example) for 3-24 hours, preferably 10-24 hours, at 10-70°C, preferably 40-60°C. The pH of the aforementioned mixed solution should be adjusted to 3-10, preferably 5-7. This adjustment is preferable because it prevents precipitation of silver oxide and such on the zeolite surface or in the fine pores. Each ion in the mixed aqueous solution is usually supplied in the form of a salt. For example, silver ions are from silver nitrate, silver sulfate, silver perchlorate, diamminesilver nitrate, diamminesilver sulfate, etc.; copper ions are from copper nitrate (II), copper perchlorate, copper acetate, potassium tetracyanocuprate, copper sulfate, etc.; zinc ions are from zinc nitrate (II), zinc sulfate, zinc perchlorate, zinc thiocyanate, zinc acetate, etc.; mercury ions are from mercury perchlorate, mercury nitrate, and mercury acetate; tin ions are from tin sulfate and such; lead ions are from lead sulfate, lead nitrate, etc.; bismuth ions are from bismuth chloride, bismuth iodide, etc.; cadmium ions are from cadmium perchlorate, cadmium sulfate, cadmium nitrate, and cadmium acetate; chromium ions are from chromium perchlorate, chromium sulfate, chromium ammonium sulfate, chromium nitrate, etc.; thallium ions are from thallium perchlorate, thallium sulfate, thallium nitrate, thallium acetate, etc.

The antibacterial metal ion content in the zeolite can be controlled by adjusting the concentration of each ion (salt) in said mixed aqueous solution. For example, in the case of antibacterial zeolite containing silver ions, an antibacterial zeolite with a silver ion content of 0.1-5% can be obtained by adjusting the silver ion concentration in said mixed aqueous solution to 0.002M/1-0.15M/1. In the case of antibacterial zeolite additionally containing copper ions and zinc ions, an antibacterial zeolite with a copper ion content of 0.1-8% and a zinc ion content of 0.1-8% can be obtained by adjusting the copper ion concentration to 0.1M/l-0.85M/l and the zinc ion concentration to 0.15M/l-1.2M/l in said mixed aqueous solution. For the ion exchange of antibacterial zeolite, it is also possible to use solutions, each of which contains each ion, and expose the zeolite with these solutions one after another. The concentration of each ion in each aqueous solution can be determined based on the concentration of each ion in said mixed aqueous solution.

After completion of the ion exchange, the zeolite is thoroughly rinsed and then dried. The drying is preferably done at 105°C -115°C, or under a reduced pressure (1-30 Torr) at 70-90°C.

Ion exchange for organic ions and/or for ions for which there isn't an adequate water soluble salt, such as tin and bismuth, can be done by using an organic solvent solution such as an alcohol or acetone to prevent precipitation of slightly soluble basic salts.

The blend ratio of the antibacterial zeolite in the external preparation for skin is not limited in particular. It is determined based on the product form of the external preparation for skin. Usually, 0.1-90 mass %, preferably 1-70 mass %, more preferably 5-70 mass % of the total amount of the external preparation for skin is blended in depending on the product form. It is blended in a similar manner for the total amount of the deodorant cosmetic as well. When the deodorant cosmetic is a stick-type cosmetic, 5-20 mass % is particularly preferable.

The average particle size of the antibacterial zeolite is preferably 10 micrometers or less. More preferably it is 0.1-5 micrometers. When the average particle size is in this range, it is preferable that 20% or less have a particle size larger than 15 micrometer in terms of the particle size distribution.

### <Nonvolatile liquid oil component>

Selection of the nonvolatile liquid oil component used in the present invention is not limited. Examples include liquid oils and fats such as linseed oil, tsubaki oil, macadamia nut oil, corn oil, olive oil, avocado oil, sasanqua oil, castor oil, safflower oil, apricot kernel oil, cinnamon oil, jojoba oil, grape oil, almond oil, rapeseed oil, sesame oil, sunflower oil, wheat germ oil, rice germ oil, rice bran oil, cotton seed oil, soybean oil, peanut oil, tea seed oil, evening primrose oil, egg yolk oil, liver oil, triglycerin, glyceryl trioctanoate, and glyceryl triisopalmitate; octanoic esters such as cetyl octanoate, isooctanoic esters such as glyceryl tri-2-ethylhexanoate and pentaerythritol tetra-2-ethylhexanoate, lauric esters such as hexyl laurate, myristic esters such as isopropyl myristate and octyldodecyl myristate, palmitic esters such as octyl palmitate, isostearic esters such as isocetyl stearate, isostearic esters such as isopropyl isostearate, isopalmitic esters such as octyl isopalmitate, oleic esters such as isodecyl oleate, adipic esters such as diisopropyl adipate, sebacic diesters such as diethyl sabacate, and ester oils such as diisostearyl malate; and liquid hydrocarbon oils such as liquid paraffin and squalane.

Furthermore, for the silicone oil, it is also possible to use chain silicones such as dimethylpolysiloxane, methylphenylpolysiloxane, and methylhydrogenpolysiloxane and liquid oils such as amino-modified silicone, polyether-modified silicone, carboxy-modified silicone, alkyl-modified silicone, ammonium salt-modified silicone, and fluorine-modified silicone.
When the external preparation for skin of the present invention is a deodorant cosmetic, in terms of usability, it is preferable to blend in nonvolatile liquid oil components such as liquid petrolatum, cetyl octanoate, methylphenylpolysiloxane, squalane, glyceryl tri-2-ethylhexanoate, glyceryl diisostearate, pentaerythrityl tetra-2-ethylhexanoate, trimethylolpropane trioctanoate, trimethylolpropane triisostearate, and diisostearyl malate.

The blend ratio of the nonvolatile liquid oil components is not limited in particular; it is preferable to blend in 5 mass % or more of the total amount of the external preparation for skin for the purpose of reducing the whiteness of the powder on the skin after application and improving usability such as adhesion to the skin, and, for the stick type, ease of application (e.g. ease of sliding). Also, for the stick type, 10 mass % or more is preferable to stably add the solid oil component for solidifying the liquid components.
There is no upper limit for the blend ratio; the maximum is about 90 mass % of the total amount of the external preparation for skin.
The significance of the present invention is that, even in the case of an external preparation for skin having a high blend ratio of a nonvolatile liquid oil component (e.g. an external preparation for skin having 10 mass % or more of it), the cleaning effect of washing on stained clothing is improved by adding a nonionic surfactant having a HLB of 6-8 that has a polyoxyethylene chain. Therefore, in the present invention, an external preparation for skin that has the nonvolatile liquid oil in the amount of 10-50 mass % of the total amount of the external preparation for skin is preferable.

The external preparation for skin of the present invention contains a nonvolatile liquid oil component. The conventional technology wherein polyoxyethylene polyoxypropylene 2-decyltetradecyl ether is added cannot sufficiently improve the cleanability of stained clothing due to the antibacterial zeolite. It is possible to attempt a reduction of stains by increasing the blend ratio of the polyoxyethylene polyoxypropylene 2-decyltetradecyl ether; but this is not preferable in terms of the sensation during use.

The present invention does not use polyoxyethylene polyoxypropylene 2-decyltetradecyl ether and instead adds a nonionic surfactant having a HLB of 6-8 that has a polyoxyethylene chain to improve the cleaning effect of washing.

### <Nonionic surfactant having a HLB of 6-8 that has a polyoxyethylene chain>

The essence of the present invention is to add a nonionic surfactant that has a polyoxyethylene chain. It is also necessary for the HLB of the nonionic surfactant that has a polyoxyethylene chain to be 6-8.
As such a nonionic surfactant, POE glycerin fatty acid ester, POE sorbitan fatty acid ester, POE sorbit fatty acid ester, POE alkyl ether, polyethylene glycol fatty acid ester, and POE trimethylolpropane fatty acid ester are preferably blended into the external preparation for skin of the present invention.
Particularly preferable are polyethylene glycol fatty acid ester (e.g. PEG-8 diisostearate (EMALEX400di-IS from Nihon Emulsion) and PEG-12 diisostearate (EMALEX600di-IS from Nihon Emulsion).
Also, POE glycerin fatty acid ester (e.g. PEG diisostearate (EMALEX GWIS320)), which has self emulsifying properties, is preferably used.

The blend ratio of the nonionic surfactant that has a polyoxyethylene chain is preferably 10 mass % or more to obtain a sufficient cleaning effect by washing.

### <Powder: Porous silica having a specific surface area of 150 m²/g or more>

In the present invention, discoloration of the external preparation's base agent itself (particularly the oil based base agent) can be prevented by further adding powder. The powder used in the present invention is preferably porous silica having a specific surface area of 150 m²/g or more. More preferable is those having a specific surface area of 300 m²/g or more.
The specific surface area is calculated by measuring the nitrogen adsorption onto the powder at 77 K and analyzing it with the BET method. The OMNISORP made by Beckmann Coluter is used as the measurement instrument.

The blend ratio of the aforementioned porous silica and such is not limited in particular. It is determined based on the product form of the external preparation for skin. Usually it is 0.1-90 mass %, preferably 1-50 mass %, more preferably 3-10 mass % of the total amount of the external preparation for skin. It is blended in a similar manner for the total amount of the deodorant cosmetic as well. When the deodorant cosmetic is a stick-type cosmetic, 3-10 mass % is particularly preferable.

Applications of the external preparation for skin of the present invention are not limited; it is preferably used as a deodorant cosmetic. In addition to the aforementioned essential ingredients, other ingredients commonly used in cosmetics, for example one, two or more of those listed below, are blended as necessary in the external preparation for skin of the present invention; the preparation can be conducted for the target formulation with a conventional method.

Solid/semisolid fats and oils, waxes, hydrocarbon oils, higher fatty acids, higher alcohols, and silicone waxes can be blended in.
Examples of the solid fats and oils include cacao butter, coconut oil, hydrogenated coconut oil, palm oil, palm kernel oil, Japanese core wax nucleus oil, Japanese core wax, and hydrogenated castor oil.

Examples of the waxes include candelilla wax, rice bran wax, beeswax, cotton wax, carnauba wax, lanolin, and shellac wax.

Examples of the hydrocarbon oils include liquid ozocerite, ceresin, polyethylene wax, microcrystalline wax, paraffin, and petrolatum.

Examples of the higher fatty acids include lauric acid, myristic acid, palmitic acid, stearic acid, behenic acid, 12-hydroxystearic acid, and undecylenic acid.

Examples of the higher alcohols include straight chain alcohols (for example, lauryl alcohol, cetyl alcohol, stearyl alcohol, behenyl alcohol, myristyl alcohol, and cetostearyl alcohol) and branched chain ethyl alcohols (for example, mono stearyl glycerin ether (batyl alcohol) ).

Examples of the solid/semisolid ester oil include myristyl myristate, cetyl myristate, stearyl stearate, cetyl stearate, cetyl palmitate, cholesteryl stearate, cholesteryl oleate, dextrin palmitate, inulin stearate and hydrogenated jojoba oil.

Examples of the silicone wax include highly polymerized methylpolysiloxanes such as highly polymerized dimethylpolysiloxane, highly polymerized methylphenylsiloxane, and highly polymerized methylvinylpolysiloxane, as well as highly polymerized amino-modified methylpolysiloxane, alkyl-modified silicone (e.g. stearyl dimethicone and alkyl (C30-C45) methicone), polyamide-modified silicone, and long chain alkoxy-modified silane (such as stearoxytrimethylsilane).

Examples of the volatile liquid oil component include cyclic dimethyl silicone (octamethylcyclotetrasiloxane, decamethylcyclopentasiloxane, and dodecamethylcyclohexasiloxane), decamethyltetrasiloxane, and octamethyltetrasiloxane.

Examples of humectants include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl-12-hydroxy stearate, sodium lactate, bile salt, dl-pyrrolidone carboxylic acid salt, short chain soluble collagen, diglycerin (EO)PO adduct, chestnut rose extract, yarrow extract, and sweet clover extract.

Examples of natural water-soluble polymers include plant-based polymers (such as gum arabic, gum tragacanth, galactan, guar gum, carob gum, karaya gum, carrageenan, pectin, agar, quince seed (Cydonia oblonga), algae colloids (brown algae extract), starches (rice, corn, potato, and wheat), and glycyrrhizic acid); microorganism-based polymers (for example, xanthan gum, dextran, succinoglucan, and pullulan); and other polymers (for example, fish collagen, fish gelatin, wheat protein, and silk protein).

Examples of the semisynthetic water-soluble polymers include: starch-type polymers (for example, carboxymethyl starch and methylhydroxypropyl starch); cellulosic polymers (for example, methyl cellulose, ethyl cellulose, methylhydroxypropyl cellulose, hydroxyethyl cellulose, cellulose sodium sulfate, hydroxypropyl cellulose, carboxymetyl-cellulose, sodium carboxymethyl cellulose, crystal cellulose, and cellulose powder); and alginic acid-type polymers (for example, sodium alginate and propyleneglycol alginate).

Examples of synthetic water-soluble polymers include vinyl polymers (for example, polyvinyl alcohol, polyvinyl methyl ether, polyvinylpyrrolidone, and carboxy vinyl polymer); polyoxyethylene polymers (for example, a copolymer of polyethylene glycol 20,000, 40,000, or 60,000 and polyoxyethylene polyoxypropylene); acrylic polymers (for example, sodium polyacrylate, polyethylacrylate, and polyacrylamide); polyethyleneimine; and cationic polymers.

Examples of thickeners include gum arabic, carrageenan, karaya gum, gum tragacanth, carob gum, quince seed (Cydonia oblonga), casein, dextrin, gelatin, sodium pectate, sodium alginate, methyl cellulose, ethyl cellulose, CMC, hydroxy ethyl cellulose, hydroxypropyl cellulose, PVA, PVM, PVP, sodium polyacrylate, carboxy vinyl polymer, locust bean gum, guar gum, tamarind gum, cellulose dialkyl dimethylammonium sulfate, xanthan gum, aluminum magnesium silicate, bentonite, hectorite, AlMg silicate (beagum), laponite, and silicic acid anhydride.

### Examples of ultraviolet absorbents include the following compounds.

### (1) Benzoic acid ultraviolet light absorbents

For example, paraminobenzoic acid (hereafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester.

### (2) Anthranilic acid ultraviolet light absorbents

For example, homo mentyl-N-acetyl anthranilate.

### (3) Salicylic acid ultraviolet light absorbents

For example, amyl salicylate, mentyl salicylate, homo mentyl salicylate, octyl salicylate, phenyl salicylate, benzyl salicylate, and p-isopropanol phenyl salicylate).

### (4) Cinnamic acid ultraviolet light absorbents

For example, octylcinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glyceryl mono-2-ethyl hexanoyl-diparamethoxycinnamate.

### (5) Triazine ultraviolet light absorbents

### Examples include bisresorsinyl triazine.

More specifically, bis{[4-(2-ethylhexyloxy)-2-hydroxy]phenyl}-6-(4-methoxyphenyl)1,3,5-triazine, and 2,4,6-tris{4-(20ethylhexyloxycarbonyl)anilino}1,3,5-triazine. 2,4-bis{[4-(2-ethylhexyloxy)-2-hydroxy]phenyl}-6-(4-methoxyphenyl)1,3,5-triazine.

### (6) Other ultraviolet light absorbents

For example, 3-(4'-methylbenzylidene)-d,1-camphor, 3-benzylidene-d,1-camphor, 2-phenyl-5-methyl benzoxazol, 2-(2'-hydroxy-5'-methylphenyl) benzotriazol, 2-(2'-hydroxy-5'-t-octylphenyl) benzotriazol, 2-(2'-hydroxy-5'-methylphenyl benzotriazol, dibenzaladine, dianisoylmethane, and 4-methoxy-4'-t-butyl dibenzoyl-methane, 5-(3,3-dimethyl-2-norbornylidene)-3-pentane-2-one. Also, pyridazine derivatives such as dimorpholinopyridazinone. Octocrylene.

Examples of the sequestering agents include: 1-hydroxy ethane-1,1-diphosphonic acid, 1-hydroxy ethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, and succinic acid.

Examples of the lower alcohols include ethanol, propanol, isopropanol, isobutyl alcohol, and t-butyl alcohol.

Examples of polyhydric alcohols include dihydric alcohols (for example, ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (for example, glycerin and trimethylolpropane); tetrahydric alcohols (for example, pentaerythritols such as 1,2,6-hexanetriol); pentahydric alcohols (for example, xylitol); hexahydric alcohols (for example, sorbitol and mannitol); polyhydric alcohol polymers (for example, diethylene glycol, dipropylene glycol, triethylene glycol, polypropylene glycol, tetraethylene glycol, diglycerin, polyethylene glycol, triglycerin, tetraglycerin, and polyglycerin); dihydric alcohol alkylethers (for example, ethylene glycol monomethyl ether, ethylene glycol monoethyl ether, ethylene glycol monobutyl ether, ethylene glycol monophenyl ether, ethylene glycol monohexyl ether, ethylene glycol mono 2-methyl hexyl ether, ethylene glycol isoamyl ether, ethylene glycol benzyl ether, ethylene glycol isopropyl ether, ethylene glycol dimethylether, ethylene glycol diethyl ether, and ethylene glycol dibutyl ether); dihydric alcohol alkylethers (for example, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); dihydric alcohol ether esters (for example, ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerin mono alkyl ethers (for example, chimyl alcohol, selachyl alcohol, and batyl alcohol); sugar alcohols (for example, sorbitol, maltitol, maltotriose, mannitol, sucrose, erythritol, glucose, fructose, starch amylolysis sugar, maltose, xylitose, and starch amylolysis sugar reduction alcohols); glysolid; tetrahydro furfuryl alcohol; P0E-tetrahydro furfuryl alcohol; POP-butyl ether; POP·POE-butyl ether; tripoli oxypropylene glycerin ether; POP-glycerin ether; POP-glycerin ether phosphoric acid; POP·POE-pentane erythritol ether, and polyglycerin.

Examples of monosaccharides include trioses (for example, D-glyceryl aldehyde and dihydroxyacetone); tetroses (for example, D-erythrose, D-erythrulose, D-threose, and erythritol); pentoses (for example, L-arabinose, D-xylose, L-lyxose, D-arabinose, D-ribose, D-ribulose, D-xylulose, and L-xylulose); hexoses (for example, D-glucose, D-talose, D-psicose, D-galactose, D-fructose, L-galactose, L-mannose, and D-tagatose); heptoses (for example, aldoheptose and heprose); octoses (for example, octurose); deoxysugars (for example, 2-deoxy-D-ribose, 6-deoxy-L-galactose, and 6-deoxy-L-mannose); amino sugars (for example, D-glucosamine, D-galactosamine, sialic acid, amino uronic acid, and muramic acid); and uronic acids (for example, D-glucuronic acid, D-mannuronic acid, L-guluronic acid, D-galacturonic acid, and L-iduronic acid).

Examples of the oligosaccharides include sucrose, umbelliferose, lactose, planteose, isolignoses, α,α-trehalose, raffinose, lignoses, umbilicine, stachyose and verbascose.

Examples of the polysaccharides include cellulose, quince seed, chondroitin sulfate, starch, galactan, dermatan sulfate, glycogen, gum arabic, heparan sulfate, hyaluronic acid, traganth gum, keratan sulfate, chondroitin, xanthan gum, mucoitin sulfuric acid, guar gum, dextran, kerato sulfate, locustbean gum, succinoglucane, and charonic acid.

Examples of amino acids include neutral amino acids (for example, threonine and cysteine) and basic amino acids (for example, hydroxylysine). Examples of the amino acid derivatives include sodium acyl sarcosinate (sodium N-lauroyl sarcosinate), acyl glutamate, sodium acyl β-alanine, glutathione, and pyrrolidone carboxylic acid.

Examples of organic amines include monoethanolamine, diethanolamine, triethanolamine, morpholine, triisopropanolamine, 2-amino-2-carbinyl-1,3-propanediol, and 2-amino-2-methyl-1-propanol.

Examples of polymer emulsions include acrylic resin emulsions, ethyl polyacrylate emulsions, acryl resin liquids, polyacrylic alkyl ester emulsions, polyvinyl acetate resin emulsions, and natural rubber latex.

Examples of pH adjustment agents include buffers such as lactic acid-sodium lactate, citric acid-sodium citrate, and succinic acid-sodium succinate.

Examples of vitamins include vitamin A, B1, B2, B6, C and E as well as their derivatives, pantothenic acid and its derivatives, and biotin.

Examples of the antioxidants include tocopherols, dibutyl hydroxytoluene, butyl hydroxyanisole, and gallic ester.

Examples of the antioxidation auxiliary agents include phosphoric acid, citric acid, ascorbic acid, maleic acid, malonic acid, succinic acid, fumaric acid, cephalin, hexameta phosphate, phytic acid, ethylenediaminetetrakis (2-hydroxyisopropyl) dioleate, ethylenediaminetetra polyoxypropylene, sodium ethylenediaminehydroxyethyl triacetate (dihydrate salt), calcium sodium ethylenediamine tetracetate, edetic acid, trisodium edetate, dipotassium edetate dihydrate, disodiemu edetate, tetrasodium edetate, tetrasodium edetate dihydrate, and tetrasodium edetate tetrahydrate.

Examples of other possible ingredients include antiseptics (methylparaben, ethylparaben, butylparaben, and phenoxyethanol); antiinflammatory agents (for example, glycyrrhizic acid derivatives, glycyrrhetinic acid derivatives, salicylic acid derivatives, hinokitiol, zinc oxide, and allantoin); whitening agents (for example, creeping saxifrage extract, arbutin, tranexamic acid, L-ascorbic acid, magnesium L-ascorbyl phosphate, L-ascorbic acid glucosie, and potassium 4-methoxysalicylate); various extracts (for example, green tea, oolong tea, black tea, puar tea, mulberry, Clara, Phellodendri Cortex, goldthread, lithospermum root , Paeonia lactiflora, Swertia japonica, Birch, sage, loquat, carrot, aloe, Malva sylvestris, Iris, grape, Coix ma-yuen, sponge gourd, lily, saffron, Cnidium officinale, sheng jiang, Hypericum erectum, 0nonis, garlic, Guinea pepper, chen pi, Ligusticum acutilobum, and seaweed), activators (royal jelly, photosensitive substances, and cholesterol derivatives); blood circulation promoting agents (for example, nonyl acid valenyl amide, nicotinic acid benzyl esters, nicotinic acid β-butoxy ethyl esters, capsaicin, gingeron, cantharis tincture, Ichthammol, tannic acid, α-borneol, tocopherol nicotinate, inositol hexanicotinate, cyclandelate, cinnarizine, tolazoline, acetylcholine, verapamil, cepharanthine, and γ-orizanol); anti-seborrhea agents (for example, sulfur and thiantol); and antiinflammatory agents (for example, thiotaurine and hypotaurine); and bactericides (for example, benzoic acid and its salts, isopropylmethyl phenol, undecylenic acid and its salts, undecylenic acid monoethanol amide, cetyltrimethyl ammonium chloride, cetylpyridinium chloride, benzalkonium chloride, benzethonium chloride, alkyldiaminoethylglycine chloride, chlorhexidine chloride, orthophenyl phenol, chlorhexidine gluconate, cresol, chloramine T, chlorxylenol, chlorcresol, chlorfenesin, chlorobutanol, 5-chloro-2-methyl-4-isothiazoline-3-one, salicylic acid and its salts, 1,3-dimethylol-5,5-dimethylhidantoin, alkylisoquinolium bromide, domiphen bromide and its salt, sorbic acid and its salts, thymol, thylum, thiram, dehydroacetic acid and its salt, triclosan, trichlorocarbanilide, paraoxybenzoic ester, parachlorphenol, halocarban, pyrogallol, phenol, hexachlorophene, 2-methyl-4-isothiazoline-3-one, NN"-Methylenebis(N'-(3-hydroxymethyl-2,5-dioxo-4-imidazolidinyl)urea), sodium layroylsarcosine, resorcin, and hinokitiol).
Examples of the powder ingredients include inorganic powders (for example, talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstic acid metal salt, magnesium, silica, barium sulfate, firing calcium sulfate (calcined gypsum), calcium phosphate, fluorine-apatite, hydroxy apatite, ceramic powder, metallic soaps (for example, zinc myristate, calcium palmitate, and aluminum stearate), and boron nitride); organic powders (for example, polyamide resin powder (nylon powder), polyethylene powder, poly methyl methacrylate powder, benzoguanamine resin powder, polytetrafluoroethylene powder, and cellulose powder); inorganic white pigments (for example, titanium dioxide and zinc oxide); inorganic red pigments (for example, iron oxide (red iron oxide) and iron titanate); inorganic brown pigments (for example, γ-iron oxide); inorganic yellow pigments (for example, yellow iron oxide and loess); inorganic black pigments (for example, black iron oxide and low oxides of titanium); inorganic purple pigments (for example, manganese violet, cobalt violet); inorganic green pigments (for example, chromium oxide, chromium hydroxide, and cobalt titanate); inorganic blue pigments (for example, ultramarine blue and Berlin blue); pearl pigment (for example, titania coated mica, titania coated bismuth oxychloride, titania coated talc, coloration titania coated mica, bismuth oxychloride, fish scale flakes); metal powder pigments (for example, aluminum powder, copper powder); organic pigments such as Zr, barium or aluminum rake (for example, organic pigments such as red 201, red 202, red 204, red 205, red 220, red 226, red 228, red 405, orange 203, orange 204, yellow 205, yellow 401 and blue 404, as well as red 3, red 104, red 106, red 227, red 230, red 401, red 505, orange 205, yellow 4, yellow 5, yellow 202, yellow 203, green 3 and blue 1; and natural colors (for example, chlorophyll and β-carotene).

When the present invention is a deodorant cosmetic, the product form is not limited in particular. Examples include the spray type, roll-on type, powder type and pressed powder type (molded powder) type, and stick type. The spray type is prepared by filling a spray container such as an aerosol can or dispenser with the ingredients as well as a propellant such as a liquefied gas and alcohol by using a conventional method. The roll-on type is prepared by filling a roll-on container with the ingredients and alcohol by using a conventional method. For the powder type and the pressed powder type, the ingredients are mixed together with powder components and oil components, and in the case of the powder type the mixture is used as is, and in the case of the pressed powder type the mixture is molded by various molding devices using a conventional method. The stick type is prepared by mixing the ingredients with oil components (solid oil and liquid oil) and filling a container with the mixture, followed by molding, using a conventional method. Products preferable for the present invention are products prepared as an oil based base agent containing wax or a gelation agent: particularly stick type deodorant products.
A product with an oil based base agent refers to a product to which water is substantially not added compared with the total amount of the external preparation for skin. When a product with an oil based base agent adheres to clothing in particular, the stain is not easy to remove; therefore the effect of the present invention is preferably manifested for such a product.

### EXAMPLES

The present invention is described in detail below by referring to Examples. The present invention is not limited to these examples. The blend ratios are in relation to the total amount and in mass-percentage units unless specified otherwise.
Commercial raw materials were used to prepare the external preparation for skin with a conventional method and the effect of the present invention was verified.

### <Cleaning/removal effect>

1: Using the formulations shown in Table 1, solid antiperspirant stick products (oil based base agent) containing various powders are prepared.
2: A prescribed amount (0.06 g/cm²) of this is applied onto a white cotton fabric (JIS standard white fabric for dyeing fastness test).
3: 0.012 g/cm² of artificial sweat (water to 100 wt%, sodium chloride 0.8 wt%, disodium phosphate dodecahydrate 0.8 wt%) was dripped on the fabric of 2.
4: The fabric of 3 is exposed to sunlight (10 minutes) and then washed with a washing machine using a conventional laundry detergent for clothing.
5: The degree of stain adhesion (discoloration) of the fabric of 3 is evaluated visually.

### <Evaluation criteria>

Evaluation was made by comparing with the aforementioned stain adhesion (discoloration) after exposure to sunlight.
⊚ : No stain adhesion (discoloration) was observed.
O: Hardly any stain adhesion (discoloration) was observed.
△ : Stain adhesion (discoloration) was observed.
X : Stain adhesion (discoloration) was definitely observed.

**[Table 1]**

| | Comparative example | | | | Examples | | |
|---|---|---|---|---|---|---|---|
| | 1 | 2 | 3 | 4 | 1 | 2 | 3 |
| Antibacterial zeolite | 10 | 10 | 10 | 10 | 10 | 10 | 10 |
| Zinc oxide | 2 | 2 | 2 | 2 | 2 | 2 | 2 |
| Aluminum potassium sulfate | 4 | 4 | 4 | 4 | 4 | 4 | 4 |
| Volatile cyclic dimethyl silicone | Balance | Balance | Balance | Balance | Balance | Balance | Balance |
| Methylphenylsiloxane | 20 | 20 | 20 | 20 | 20 | 20 | 20 |
| Polyethylene wax | 10 | 10 | 10 | 10 | 10 | 10 | 10 |
| Sorb i tan sesquiisostearate (HLB=5) | - | 2 | - | - | - | - | - |
| PEG-4 Diisostearate (HLB=4) | - | - | 2 | - | - | - | - |
| PEG-6 Diisostearate (HLB=5) | - | - | - | 2 | - | - | - |
| PEG-8 Diisostearate (HLB=6) | - | - | - | - | 2 | - | - |
| PEG-12 Diisostearate (HLB=8) | - | - | - | - | - | 2 | - |
| PEG-20 glyceryl triisostearate (HLB=8) | - | - | - | - | - | - | 2 |
| Cleaning/removing effect | × | △ | △ | △ | ○ | ○ | ⊚ |

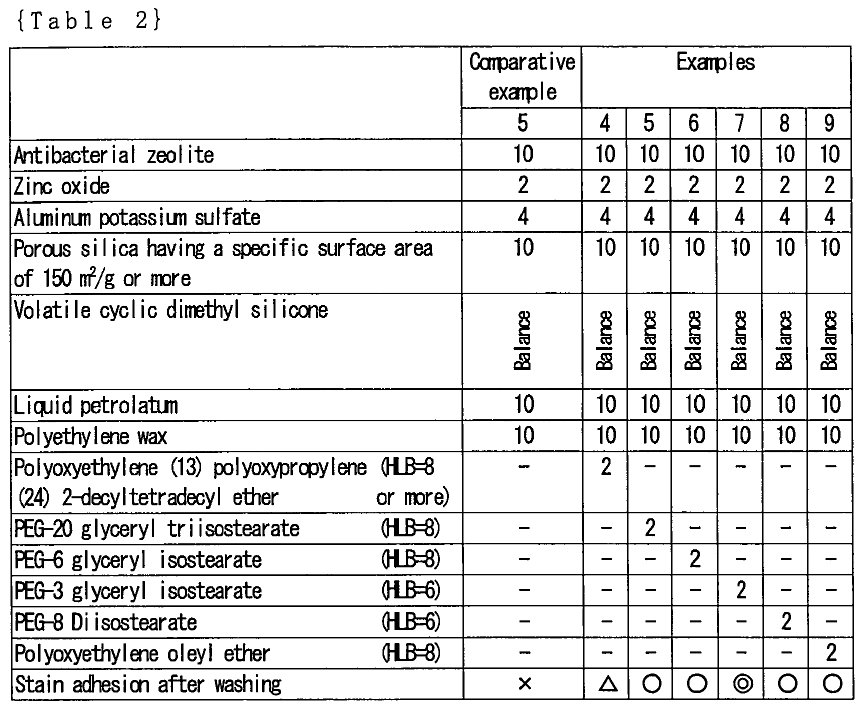

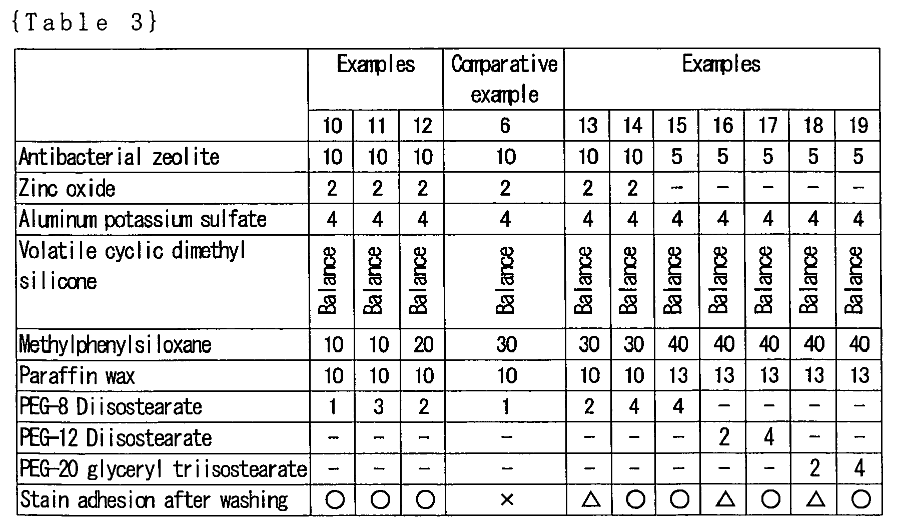

The nonvolatile liquid oil components are methylphenylpolysiloxane and liquid petrolatum.
The nonionic surfactants are sorbitan sesquiisostearate, PEG-4 to 12 diisostearate, and PEG-20 glyceryl triisostearate. Among these, those that have a polyoxyethylene chain are PEG-4 to 12 diisostearate, PEG-20 glyceryl triisostearate, polyoxyethylene (13) polyoxypropylene (24) 2-decyltetradecyl ether, and PEG (5-3) isostearate, and polyoxyethylene (5) oleyl ether. "Commercial raw materials used"
Antibacterial zeolite: Zeolite containing silver ions, zinc ions, and ammonium ions (Zeomic AJ10N from Sinanen Zeomic Co., Ltd., average particle size approximately 1.5 micrometers)
Zinc oxide: Fine zinc oxide from Sakai Chemical Industry Co. Ltd.
Aluminum potassium sulfate: Taiace K20 from Taimei Chemicals Co., Ltd.
Porous silica having a specific surface area of 150 m²/g or more: Sunsphere L-51 from Asahi Glass Co., Ltd.
Volatile cyclic dimethyl silicone: KF-995 from Shin-Etsu Chemical Co., Ltd. Methylphenylpolysiloxane: KF56 from Shin-Etsu Chemical Co., Ltd.
Polyethylene wax: PERF0RMALENE PL Polyethylene from New Phase Technologies
Paraffin wax: Refined paraffin wax from Nikko Rica Co. Ltd.
PEG-4 diisostearate: EMALEX 200di-IS from Nihon Emulsion Co., Ltd.
PEG-6 diisostearate: EMALEX 300di-IS from Nihon Emulsion Co., Ltd.
PEG-8 diisostearate: EMALEX 400di-IS from Nihon Emulsion Co., Ltd.
PEG-12 diisostearate: EMALEX 600di-IS from Nihon Emulsion Co., Ltd.
Sorbitan sesquiisostearate: Cosmol 182V from Nisshin Oillio Group Co., Ltd.
PEG-20 glyceryl triisostearate: EMALEX GWIS-320 from Nihon Emulsion Co., Ltd.
PEG-3 glyceryl isostearate: EMALEX GWIS-103 from Nihon Emulsion Co., Ltd.
PEG-6 glyceryl isostearate: EMALEX GWIS-106 from Nihon Emulsion Co., Ltd.
Polyoxyethylene (5) oleyl ether: EMALEX 505 (H) from Nihon Emulsion Co., Ltd.

These results show that Examples containing specific nonionic surfactants exhibit a cleaning/removal effect of stains by washing. Comparative examples do not show a sufficient cleaning/removal effect.
Also, for all Examples, the sensation during use does not worsen and stays excellent when the nonionic surfactant is added.
Examples 4-9 show that, when the blend ratio of the antibacterial zeolite is 10 wt%, the cleaning effect of the present invention manifests more prominently if the blend ratio of the specific nonionic surfactant is 10 wt% or more of the total amount of the nonvolatile liquid oil component.

Deodorant cosmetics (antiperspirant cosmetics) that are the external preparation for skin of the present invention are listed below. Each of these cosmetics has a superior cleaning/removal effect of stains on clothing and gives a superior sensation during use.

### Deodorant stick

**{Table 4}**

| | | Examples | | | |
|---|---|---|---|---|---|
| | | 20 | 21 | 22 | 23 |
| Powder | Antibacterial zeolite | 10 | 10 | 5 | 3 |
| | Methyl polyacrylate | | | 5 | |
| | Porous silica | | | 5 | 10 |
| | Talc | 10 | | | |
| | Zinc oxide | | 2 | | |
| | Aluminum hydroxychloride | 20 | | | |
| | Aluminum potassium sulfate | | | 4 | |
| | Red iron oxide | 0.001 | | | |
| | Yellow iron oxide | 0.01 | | | |
| 0il components | Synthesized isoparaffin | 30 | | | |
| | Methylphenylsiloxane | | | | 20 |
| | Cetyl octanoate | | 10 | | |
| | Volatile cyclic dimethyl silicone | 18.99 | 56 | 46.9 | 45 |
| | Glyceryl triethylhexanoate | | | 20 | |
| | Squalane | | 10 | | |
| | Polyethylene wax | | | 9 | |
| | Paraffin wax | 8 | | | |
| | Silicone wax | | | | 20 |
| | Hydrogenated jojoba oil | | | 3 | |
| | Stearyl alcohol | | 10 | | |
| Surfactant | PEG-8 Diisostearate | | | 2 | |
| | PEG-12 Diisostearate | | | | 2 |
| | PEG-20 glyceryl triisostearate | 3 | | | |
| | POE (5) oleyl ether | | 2 | | |
| Perfume | Perfume | | | 0.1 | 0.5 |

Using a conventional method, the aforementioned oil component and the surfactant are heated and mixed, to which the powder was added, followed by thorough dispersion with a homomixer. This is put into a stick container and cooled down to obtain a deodorant stick.

### Multi-layer type deodorant lotion

**{Table 5}**

| | | Examples | |
|---|---|---|---|
| | | 24 | 25 |
| Powder | Antibacterial zeolite | 5 | 10 |
| | Porous silica | 2 | |
| | Talc | | 2 |
| Water soluble Ingredient | Zinc para-phenol sulfonate | | 0.5 |
| | Alcohol | 47.5 | 15 |
| | Water | | 51 |
| | Dipropylene glycol | | 5 |
| Oil components | Volatile cyclic dimethyl silicone | 40 | |
| | Methylphenylsiloxane | 5 | 15 |
| Surfactant | PEG-20 glyceryl triisostearate | 0.5 | |
| | PEG-8 glyceryl isostearate | | 1.5 |

It is obtained by mixing the aforementioned ingredients using a homomixer.

### Deodorant spray

**{Table 6}**

| | | Examples | | |
|---|---|---|---|---|
| | | 26 | 27 | 28 |
| Powder | Antibacterial zeolite | 1 | 0.5 | 0.3 |
| | Aluminum hydroxychloride | 2 | | 2 |
| | Zinc oxide | 1 | 0.2 | |
| | Aluminium potassium sulfate | | 0.5 | |
| | Spherical polyethylene powder | | 0.5 | |
| | Porous silica | | 1 | 0.5 |
| | Starch powder | 1.5 | 3 | 2 |
| Oil components | Cetyl octanoate | 2 | | |
| | Synthesized isoparaffin | | 1 | |
| | Methylphenylsiloxane | 2 | 3 | 2 |
| | Volatile cyclic dimethyl silicone | | | 3 |
| Surfactant | PEG-12 dioleate | 0.4 | | |
| | POE (6) sorbit tetraoleate | | 0.3 | |
| | PEG-20 glyceryl triisostearate | | | 0.2 |
| Propellant | Isopentane | 10 | 10 | 10 |
| | Liquefied petroleum gas | 80 | 80 | 80 |

Using a conventional method, the powder parts are mixed using a kneader and the oil components are mixed using a blender. Each is sequentially put into a spray can and then the propellant is added to obtain a powder spray.

### INDUSTRIAL APPLICABILITY

The external preparation for skin of the present invention enables effective removal of its stain on clothing and exhibits a superior deodorizing effect. Therefore it is preferably used in particular as a deodorant cosmetic.

## Claims

1. An external preparation for skin comprising antibacterial zeolite and a nonvolatile liquid oil component, said external preparation for skin comprising a nonionic surfactant having a HLB of 6-8 that has a polyoxyethylene chain.

2. The external preparation for skin of claim 1 wherein the blend ratio of said nonionic surfactant having a HLB of 6-8 that has a polyoxyethylene chain is 10 mass % or more of the total amount of the nonvolatile liquid oil component.

3. The external preparation for skin of claim 1 or 2 that additionally comprises porous silica having a specific surface area of 150 m²/g or more.

4. The external preparation for skin of any of claims 1-3 wherein said nonvolatile liquid oil component is one, two or more selected from a group consisting of liquid petrolatum, cetyl octanoate, methylphenylpolysiloxane, squalane, glyceryl tri-2-ethylhexanoate, glyceryl diisostearate, pentaerythrityl tetra-2-ethylhexanoate, trimethylolpropane trioctanoate, trimethylolpropane triisostearate, and diisostearyl malate.

5. The external preparation for skin of any of claims 1-4 wherein the external preparation for skin is prepared as an oil based product.

6. The external preparation for skin of any of claims 1-5 wherein the external preparation for skin is in the stick form.
